# EUROPEAN PATENT APPLICATION

(11) **EP 4 799 743 A1**
(43) Date of publication of application: **02.09.2026**
(21) Application number: 26159524.3
(22) Date of filing: 19.02.2026
(51) Int. Cl.: B06B 1/06

(54) **ULTRASOUND PROBE**

(30) Priority: 26.02.2025 JP 2025028899
(71) Applicant: NOK Corporation, Minato-ku Tokyo 105-8585 (JP)
(72) Inventor: SATO, Kosuke, Fujisawa-shi, Kanagawa 2510042 (JP); UDA, Toru, Fujisawa-shi, Kanagawa 2510042 (JP)
(74) Representative: TBK

(57) **Abstract**

An ultrasound probe includes a piezoelectric body including a first surface; an insulating layer including a covering portion that covers a portion of the first surface and a peripheral edge portion that in plan view does not overlap the piezoelectric body; a first electrode including an electrode portion in contact with the first surface and a terminal portion in contact with a surface of the peripheral edge portion; and a wiring substrate bonded to the terminal portion of the first electrode and to the peripheral edge portion of the insulating layer.

## Description

### CROSS REFERENCE TO RELATED APPLICATION

This Application is based on and claims priority from Japanese Patent Application No. 2025-028899, filed on February 26, 2025, the entire content of which is incorporated herein by reference.

### BACKGROUND

### Technical Field

The present disclosure relates to ultrasound probes.

### Description of Related Art

An ultrasound probe that transmits and receives ultrasound waves with a piezoelectric body interposed between a first electrode and a second electrode constituting a laminate structure is known in the art. For example, International Publication No. WO 2023/140166 discloses an ultrasound probe having a piezoelectric body formed using a sol-gel method, and having an upper electrode in contact with an upper surface of the piezoelectric body, and a lower electrode in contact with a lower surface of the piezoelectric body. A flexible wiring substrate for application of a voltage to the upper electrode is bonded to an end portion of the upper electrode on the surface of the piezoelectric body.

In a configuration in which the wiring substrate is bonded to the upper electrode on the surface of the piezoelectric body, an external force acting on the wiring substrate may transfer in a thickness direction of the piezoelectric body and act on the upper electrode and the piezoelectric body. The external force acting on the piezoelectric body from the wiring substrate may cause defects such as peeling of the piezoelectric body.

### SUMMARY

In view of the foregoing, an object of one aspect of the present disclosure is to reduce occurrence of defects, such as peeling of a piezoelectric body caused by an external force acting on the piezoelectric body via the wiring substrate.

An ultrasound probe according to an aspect of the present disclosure includes: a piezoelectric body including a first surface; an insulating layer including a covering portion that covers a portion of the first surface, and a peripheral edge portion that, in plan view, does not overlap the piezoelectric body; a first electrode including an electrode portion in contact with the first surface, and a terminal portion in contact with a surface of the peripheral edge portion; and a wiring substrate bonded to the terminal portion of the first electrode and to the peripheral edge portion of the insulating layer.

### BRIEF DESCRIPTION OF THE DRAWINGS

Fig. 1 is a plan view of an ultrasound probe according to the embodiment.
Fig. 2 is a cross-sectional view taken along line II-II in Fig. 1.
Fig. 3 is an enlarged plan view of a plurality of first electrodes.
Fig. 4 is a cross-sectional view of an ultrasound probe according to a comparative example.
Fig. 5 is a process diagram showing steps of manufacturing an ultrasound probe.

### DETAILED DESCRIPTION OF THE EMBODIMENTS

An embodiment of the present disclosure will now be described with reference to the drawings. In the drawings, dimensions and scales of constituent parts may differ from those of actual products. Furthermore, in the embodiment described below an exemplary form is disclosed for implementing the present disclosure. Accordingly, the scope of the present disclosure is not limited to the exemplary form disclosed in the description in the following embodiment.

### A: Embodiment

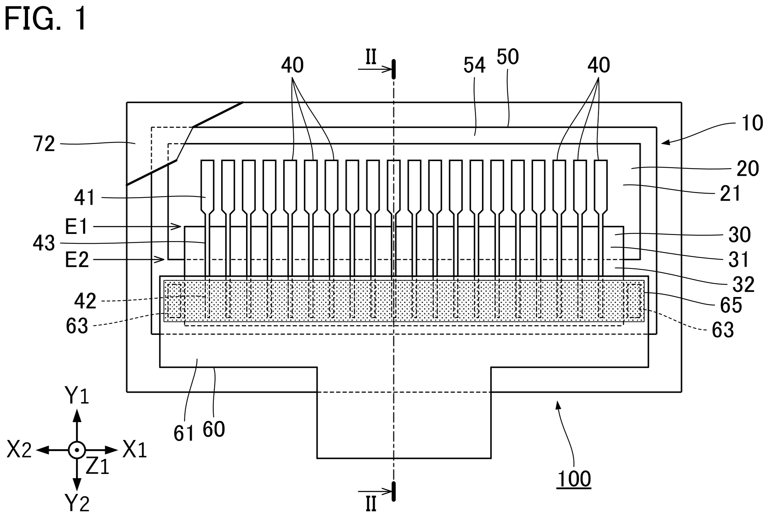

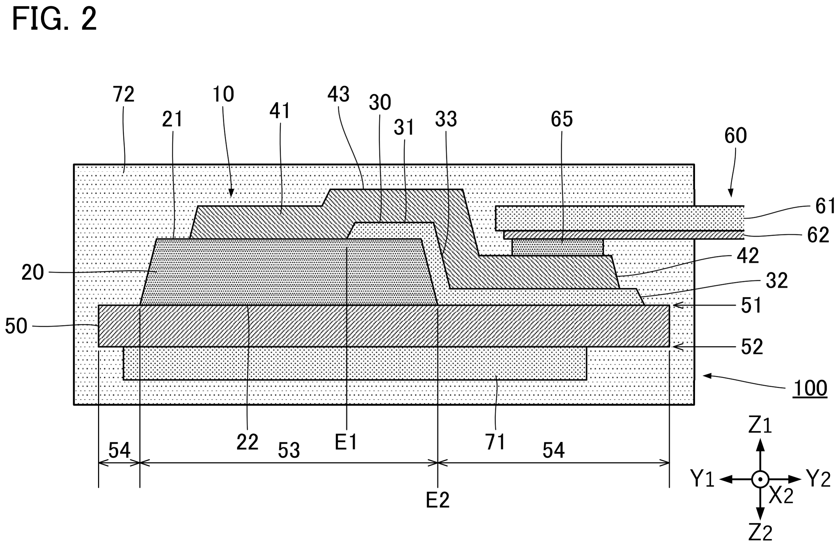

Fig. 1 is a plan view of an ultrasound probe 100 according to one embodiment of the present disclosure. Fig. 2 is a cross-sectional view taken along line II-II in Fig. 1. In the following description, three axes (an X-axis, Y-axis, and Z-axis), each orthogonal to each other, are assumed. One direction along the X-axis is referred to as an X1 direction, and a direction opposite to the X1 direction along the X-axis is referred to as an X2 direction. Similarly, one direction along the Y-axis is referred to as a Y1 direction and a direction opposite to the Y1 direction along the Y axis is referred to as a Y2 direction; and, one direction along the Z axis is referred to as a Z1 direction and a direction opposite to the Z1 direction along the Z axis is referred to as a Z2 direction. Furthermore, an element of the ultrasound probe 100 viewed from a line of sight along the Z-axis is referred to as being in "plan view."

The ultrasound probe 100 of the present embodiment is used for ultrasound measurement within a living body for medical observation. Ultrasound measurement is a method of observing an internal structure of a living body. More specifically, ultrasound waves are transmitted from the ultrasound probe 100 into the living body, and the ultrasound probe 100 receives emitted ultrasound waves reflected from within the living body. By carrying out the ultrasound measurement by use of the ultrasound probe 100, an internal structure of the living body can be inspected in a non-invasive manner.

The ultrasound probe 100 is flexible, and able to closely conform to a curved surface of a living body, such as a neck, chest, abdomen, arm, wrist, or finger. As illustrated in Figs. 1 and 2, the ultrasound probe 100 includes a piezoelectric unit 10, a wiring substrate 60, a support member 71, and a protective cover 72. For convenience of illustration, Fig. 1 shows a portion of the protective cover 72 of the piezoelectric unit 10 cut away in the Z1-direction.

The piezoelectric unit 10 is an inspection unit that is used for transmitting and receiving ultrasound waves. The piezoelectric unit 10 converts electrical drive signals of varying voltages into ultrasound waves, and converts received ultrasound waves into electrical signals of varying voltages. The piezoelectric unit 10 transmits ultrasound waves in the Z1 direction and receives ultrasound waves received from the Z1 direction. As illustrated in Figs. 1 and 2, the piezoelectric unit 10 includes a piezoelectric body 20, an insulating layer 30, a plurality of first electrodes 40, and a second electrode 50.

The piezoelectric body 20 is a structure that converts electrical drive signals of varying voltages into mechanical vibrations, and converts mechanical vibrations into electrical signals of varying voltages by piezoelectric effect. The piezoelectric body 20 is formed of, for example, a piezoelectric material such as lead zirconate titanate (PZT). The piezoelectric body 20 is formed of a flexible porous material that is deformable when subject to an external force. The method of manufacturing the piezoelectric body 20 is not particularly limited. For example, the piezoelectric body 20 may be formed by a sol-gel method. That is, the piezoelectric body 20 may be formed by the sol-gel method to provide a sol-gel film. Formation of the piezoelectric body 20 by the sol-gel method is disclosed in, for example, Japanese Patent Publication No. 7092277. The piezoelectric body 20 is constituted of stacked layers that are provided by repeating a film forming process, for example. The film thickness of the piezoelectric body 20 is, for example, 10 µm or more and 200 µm or less (more preferably, 50 µm or more and 100 µm or less). The manufacturing method and the dimensions of the piezoelectric body 20 are not limited to the above examples and may be modified as appropriate. Further, the piezoelectric body 20 is not limited to a porous material. Any material that has a piezoelectric effect and is flexible can be used as the piezoelectric body 20.

As seen in plan view, the piezoelectric body 20 is a plate-shaped member with a rectangular shape elongated in the X-axis direction. The piezoelectric body 20 has a first surface 21 and a second surface 22 that are parallel to an X-Y plane. The first surface 21 is a main surface of the piezoelectric body 20 that faces in the Z1 direction. The second surface 22 is a main surface of the piezoelectric body 20 that faces in the Z2 direction. That is, the second surface 22 of the piezoelectric body 20 opposes the first surface 21 of the piezoelectric body 20.

The second electrode 50 is a flat conductive member (lower electrode) disposed in the Z2 direction of the piezoelectric body 20. As seen in plan view, the second electrode 50 has a rectangular shape elongated in the direction of the X-axis. The second electrode 50 is formed of a metal such as stainless steel. The second electrode 50 has a thickness sufficient to provide adequate mechanical strength and flexibility. The thickness of the second electrode 50 is, for example, 10 µm or more and 200 µm or less (more preferably, 50 µm or more and 100 µm or less).

The second electrode 50 includes a front surface 51 and a rear surface 52 that are parallel to the X-Y plane. The front surface 51 is a main surface of the second electrode 50 and faces in the Z1 direction. The rear surface 52 is a main surface of the second electrode 50 and faces in the Z2 direction. The piezoelectric body 20 is formed on a portion of the front surface 51 of the second electrode 50. That is, as seen in plan view, the piezoelectric body 20 and the second electrode 50 overlap each other.

As illustrated in Figs. 1 and 2, the second electrode 50 includes an electrode portion 53 and an overhang portion 54 in plan view. The electrode portion 53 overlaps the piezoelectric body 20 in plan view, and is in contact with the second surface 22 of the piezoelectric body 20. In contrast, the overhang portion 54 extends beyond the peripheral edge of the piezoelectric body 20 and does not overlap the piezoelectric body 20 in plan view. Specifically, the overhang portion 54 forms a rectangular frame that surrounds the electrode portion 53 in plan view.

The insulating layer 30 is a film body formed of an insulating material such as a resin material. The insulating layer 30 is formed on the Z1-direction side of the piezoelectric body 20 and the second electrode 50. Specifically, the insulating layer 30 extends continuously from the first surface 21 of the piezoelectric body 20 to the front surface 51 of the second electrode 50. The resin material used to form the insulating layer 30 is, for example, acryl resin, polyvinyl chloride (PVC), polyethylene (PE), polyurethane (PU), or silicone resin. Although the method of manufacturing the insulating layer 30 is not particularly limited, the insulating layer 30 may be provided as an insulating sheet of a predetermined planar shape that is attached to the first surface 21 of the piezoelectric body 20 and the front surface 51 of the second electrode 50. As stated, the method of manufacturing the insulating layer 30 is not particularly limited. For example, the insulating layer 30 may be formed by applying and curing an insulating material using a variety of printing techniques. The insulating layer 30 may be provided by attaching an insulating sheet and applying an insulating material.

The thickness of the insulating layer 30 is, for example, 1 µm or more and 100 µm or less (more preferably, 5 µm or more and 20 µm or less). The thickness of the insulating layer 30 is less than the thickness of the piezoelectric body 20. However, the film thickness of the insulating layer 30 may exceed the film thickness of the piezoelectric body 20, or the film thickness of the insulating layer 30 may be equivalent to the film thickness of the piezoelectric body 20.

The insulating layer 30 includes a covering portion 31, a peripheral edge portion 32, and a stepped portion 33. The covering portion 31, the peripheral edge portion 32, and the stepped portion 33 are continuous with one another. That is, the covering portion 31, the peripheral edge portion 32, and the stepped portion 33 together form a single contiguous film body.

The covering portion 31 covers a portion of the first surface 21 of the piezoelectric body 20. Specifically, the covering portion 31 covers a portion of the vicinity of the peripheral edge E2, which extends along the X-axis, in a Y2-direction of the first surface 21 of the piezoelectric body 20. As described above, the covering portion 31 of the insulating layer 30 overlaps the piezoelectric body 20 in plan view.

The peripheral edge portion 32 is provided on the overhang portion 54 of the second electrode 50. That is, the peripheral edge portion 32 overlaps the overhang portion 54 of the second electrode 50 in plan view. Specifically, the peripheral edge portion 32 covers an elongated region on the front surface 51 of the overhang portion 54 that extends along the X-axis in the Y2 direction. As described above, the peripheral edge portion 32 of the insulating layer 30 is in contact with the overhang portion 54 of the second electrode 50. Also as described above, the overhang portion 54 of the second electrode 50 extends in the Y2 direction beyond the peripheral edge E2 of the piezoelectric body 20 in plan view. Thus, the peripheral edge portion 32 of the insulating layer 30 does not overlap the piezoelectric body 20 in plan view. As described above, according to the configuration in which the peripheral edge portion 32 of the insulating layer 30 is in contact with the overhang portion 54 of the second electrode 50, the peripheral edge portion 32 of the insulating layer 30 is supported by the second electrode 50.

As will be understood from the above description, the peripheral edge portion 32 is disposed further in the Z2 direction than the covering portion 31. As illustrated in Fig. 2, the stepped portion 33 of the insulating layer 30 connects the covering portion 31 and the peripheral edge portion 32. Specifically, the stepped portion 33 covers the side surface (peripheral edge E2) of the piezoelectric body 20 between the covering portion 31 and the peripheral edge portion 32 in plan view.

There is a plurality of first electrodes 40 each of which is plate-shaped (an upper electrode) and is positioned in the Z1 direction relative to the piezoelectric body 20 and the second electrode 50. Each first electrode 40 is made of a low-resistance conductive material, such as copper or silver. Although the method of manufacturing the first electrodes 40 is not particularly limited, they may be provided, for example, by applying and curing a silver paste by use of a printing technique such as screen printing. The conductive material of the first electrodes 40 may be modified as appropriate. For example, the first electrodes 40 may be made of various electro-conducting elastomers, with electro-conducting filters dispersed in elastic materials, such as epoxy resins, acrylic resins, urethane resins, polyester resins, cellulose resins, silicon rubber, or urethane rubber.

The thickness of the first electrodes 40 is, for example, 5 µm or more and 50 µm or less (more preferably, 10 µm or more and 20 µm or less). The film thickness of each of the first electrodes 40 is greater than the film thickness of the insulating layer 30. However, the film thickness of the first electrodes 40 may be less than the film thickness of the insulating layer 30, or the film thickness of the first electrodes 40 may be equivalent to the film thickness of the insulating layer 30.

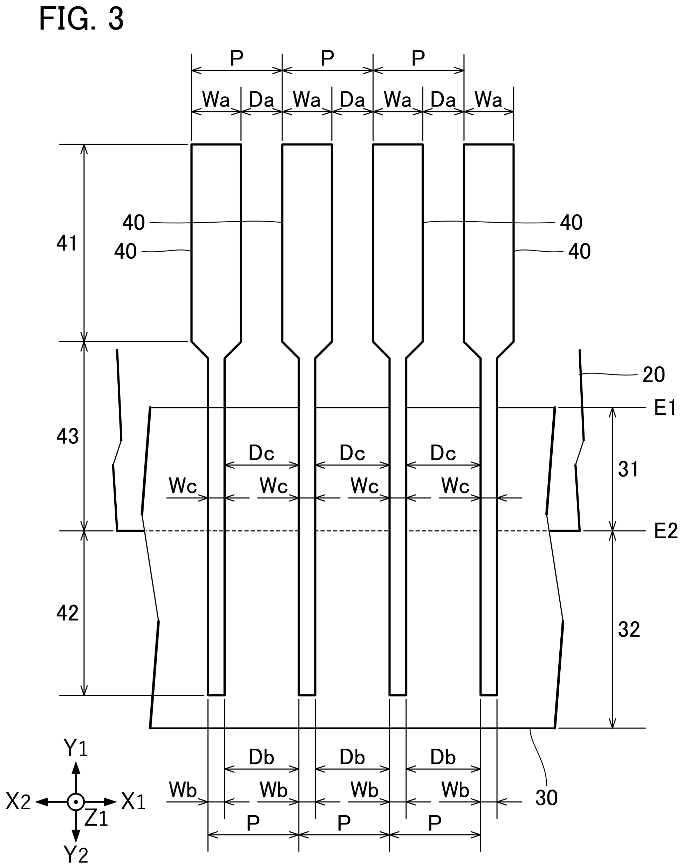

Fig. 3 is an enlarged plan view of the first electrodes 40. As illustrated in Figs. 1 and 3, the first electrodes 40 are arranged apart from one another along the X-axis in plan view. A pitch at which each of the first electrodes 40 is arranged (hereinafter, referred to as "arrangement pitch P") is constant. For example, the arrangement pitch P is 0.1 mm or more and 10.0 mm or less (more preferably, 0.5 mm or more and 1.0 mm or less). The piezoelectric body 20 is a single film body provided continuously along the X-axis over the first electrodes 40. Similarly, the insulating layer 30 is a single film body provided continuously along the X-axis over the first electrodes 40. The X-axis can also be expressed as an axial line that extends along the direction in which the first electrodes 40 are arranged.

As seen in plan view, each of the first electrodes 40 has an elongated shape extending along the Y-axis, and each of the first electrodes 40 includes an electrode portion 41, a terminal portion 42, and a connection portion 43. The electrode portion 41, the terminal portion 42, and the connection portion 43 constitute a single contiguous conductive film. The planar shapes of each of the first electrodes 40 are the same.

As seen in plan view, the electrode portion 41 of each of the first electrodes 40 overlaps the first surface 21 of the piezoelectric body 20. Specifically, the electrode portion 41 is provided on a region of the first surface 21 that is not covered with the insulating layer 30. Accordingly, the electrode portion 41 is in contact with the first surface 21 of the piezoelectric body 20. The electrode portion 41 has an elongated shape along the Y-axis.

As described above, the piezoelectric body 20 is interposed between the electrode portion 41 of the first electrode 40 and the electrode portion 53 of the second electrode 50 to constitute a laminate structure. The portion with the electrode portion 41 of the first electrode 40, the piezoelectric body 20, and the electrode portion 53 of the second electrode 50 stacked in the Z-axis direction serves as a piezoelectric element that transmits and receives ultrasound waves. That is, displacements (vibrations) corresponding to voltages between the electrode portion 41 and the electrode portion 53 are generated in the piezoelectric body 20. The Z-axis can also be expressed as an axial line along a direction in which the electrode portion 41, the piezoelectric body 20, and the electrode portion 53 are stacked.

The terminal portion 42 of each of the first electrodes 40 is a portion in contact with the surface of the peripheral edge portion 32 of the insulating layer 30. As described above, the peripheral edge portion 32 of the insulating layer 30 does not overlap the piezoelectric body 20 in plan view. Accordingly, the electrode portions 41 of the first electrodes 40 each overlap the piezoelectric body 20 in plan view, whereas the terminal portions 42 of the first electrodes 40 do not each overlap the piezoelectric body 20 in plan view. As will be understood from the above description, a stacked structure in which the terminal portion 42 of the first electrode 40, the peripheral edge portion 32 of the insulating layer 30, and the overhang portion 54 of the second electrode 50 are stacked in the Z-axis direction is formed in a region that does not overlap the piezoelectric body 20 in plan view.

The connection portion 43 of each of the first electrodes 40 is a portion that connects the electrode portion 41 and the terminal portion 42. As seen in plan view, the connection portion 43 extends from the first surface 21 of the piezoelectric body 20, via the covering portion 31 and the stepped portion 33 of the insulating layer 30 to the peripheral edge portion 32. A portion of the connection portion 43 that reaches the peripheral edge portion 32 is connected to the terminal portion 42. Accordingly, as illustrated in Figs. 1 to 3, the connection portion 43 of each of the first electrodes 40 overlaps the peripheral edge E1 of the insulating layer 30 located on the first surface 21 of the piezoelectric body 20 and the peripheral edge E2 of the piezoelectric body 20 that is covered by the insulating layer 30 as seen in plan view.

As illustrated in Fig. 3, a line width Wb of the terminal portion 42 of each of the first electrodes 40 is smaller than a line width Wa of the electrode portion 41 of each of the first electrodes 40 (Wb < Wa). The line width Wa is a dimension of the electrode portion 41 along the X-axis. The line width Wb is a dimension of the terminal portion 42 along the X-axis. For example, in a configuration in which the arrangement pitch P is 1.0 mm, the line width Wa of the electrode portion 41 is, for example, 500 µm or more and 950 µm or less (more preferably, 800 µm or more and 900 µm or less). Since the arrangement pitch P of the first electrodes 40 is constant, the spacing Db between the terminal portions 42 of first electrodes 40 adjacent to each other in the X-axis direction is greater than the spacing Da between the electrode portions 41 (Db > Da). The spacing Da between the electrode portions 41 is, for example, 50 µm or more and 500 µm or less (more preferably, 100 µm or more and 200 µm or less). On the other hand, the spacing Db between the terminal portions 42 is, for example, 100 µm or more and 900 µm or less (more preferably, 400 µm or more and 600 µm or less).

In addition, a line width Wc of the connection portion 43 of each of the plurality of first electrodes 40 is smaller than the line width Wa of the electrode portion 41 each of the first electrodes 40 (Wc < Wa). The line width Wc of the connection portion 43 is a dimension of the connection portion 43 along the X-axis. The line width Wb of the terminal portion 42 and the line width Wc of the connection portion 43 are substantially the same (Wb = Wc). As described above, since the arrangement pitch P of the first electrodes 40 is constant, the spacing Dc between the connection portions 43 of the first electrodes 40 adjacent to each other in the X-axis direction is greater than the spacing Da between the electrode portions 41 (Dc > Da). The spacing Db between the terminal portions 42 and the spacing Dc between the connection portions 43 are substantially the same (Db = Dc). For example, in a configuration in which the arrangement pitch P is 1.0 mm, the spacing Dc between the connection portions 43 is, for example, 100 µm or more and 900 µm or less (more preferably, 400 µm or more and 600 µm or less).

In a configuration in which the spacing D (Da,Db,Dc) between the first electrodes 40 is too small, the first electrodes 40 adjacent to each other may be susceptible to short-circuiting. In addition, in a configuration in which the spacing Da between the respective electrode portions 41 is too large, sufficient sensitivity of the ultrasound probe 100 may not be attained. With the spacing D within the range exemplified in the foregoing, it is possible to both reduce the occurrence of short-circuiting of the first electrodes 40 and to attain sufficient sensitivity at a high level. However, the dimensions and relative size relationships of the respective line widths W (Wa,Wb,Wc) and the respective spacings D (Da,Db,Dc) are not limited to the above examples, and may be modified as appropriate.

The wiring substrate 60 in Figs. 1 and 2 is a mounted component that is used for transmitting drive signals supplied from an external device to the piezoelectric unit 10. The wiring substrate 60 includes an insulating substrate 61, wiring 62, and wiring 63. In Fig. 1, the wiring 62 is not shown.

The insulating substrate 61 is a flexible, layered or film-like flat sheet material. That is, the wiring substrate 60 is an elastically deformable flexible wiring substrate (FPC: Flexible Printed Circuit). The wiring 62 and the wiring 63 are each conductive patterns provided on a surface of the insulating material that faces the Z2 direction. As illustrated in Fig. 1, the wiring 63 includes first wiring and second wiring, and the first wiring and the second wiring of the wiring 63 are disposed in the vicinity of the respective end portions of the insulating substrate 61 in the X-axis direction. The wiring 62 is located between the first wiring and the second wiring of the wiring 63 in plan view.

An end portion of the wiring substrate 60 is bonded to the piezoelectric unit 10. For example, an anisotropic conductive film 65 (ACF: Anisotropic Conductive Film) is used to bond the wiring substrate 60 to the piezoelectric unit 10. The anisotropic conductive film 65 is an adhesive within which a large number of conductive particles is dispersed. In Fig. 1, for convenience of reference, the anisotropic conductive film 65 is shaded.

The wiring substrate 60 is bonded to the terminal portion 42 of each of the first electrodes 40 and the peripheral edge portion 32 of the insulating layer 30 of the piezoelectric unit 10. That is, the insulating substrate 61 of the wiring substrate 60 is bonded to the peripheral edge portion 32 of the insulating layer 30 by adhesion of the anisotropic conductive film 65. The terminal portions 42 each of the first electrodes 40 and corresponding wiring 62 of the wiring substrate 60 are electrically connected to each other by the conductive particles of the anisotropic conductive film 65. Specifically, as seen in plan view, the wiring substrate 60 is installed such that the end portion of the wiring substrate 60 overlaps the peripheral edge portion 32 of the insulating layer 30. In other words, the wiring substrate 60 does not overlap, in plan view, (i) the electrode portion 41 or the connection portion 43 of each of the first electrodes 40, or (ii) the covering portion 31 or the stepped portion 33 of the insulating layer 30. That is, the wiring substrate 60 is not directly bonded to the electrode portion 41 of each of the first electrodes 40 provided on the first surface 21 of the piezoelectric body 20. The wiring 63 in Fig. 1 is bonded to the second electrode 50 by the anisotropic conductive film 65. Each wiring 63 is grounded, for example.

The support member 71 is a backing material installed on the rear surface 52 of the second electrode 50. Specifically, as seen in plan view, the support member 71 is a plate-like member that overlaps the entire area of the piezoelectric body 20. The support member 71 is made of an elastic material such as butyl rubber or silicone rubber. The support member 71 attenuates ultrasound waves radiated from the piezoelectric body 20 in the Z2 direction. The support member 71 may be omitted.

The protective cover 72 is a structure that protects the piezoelectric unit 10. Specifically, the protective cover 72 is an insulating housing that covers the entire piezoelectric unit 10 (the piezoelectric body 20, the insulating layer 30, the first electrodes 40, and the second electrode 50) and the support member 71. A part of the wiring substrate 60 in the vicinity of the end portion bonded to the piezoelectric unit 10 is accommodated in the protective cover 72, and the remaining portion of the wiring substrate 60 is exposed beyond the protective cover 72. The protective cover 72 is made of an elastic material such as silicone rubber or urethane rubber. The protective cover 72 protects the piezoelectric body 20, the insulating layer 30, the first electrode 40, and the second electrode 50. The protective cover 72 may be omitted.

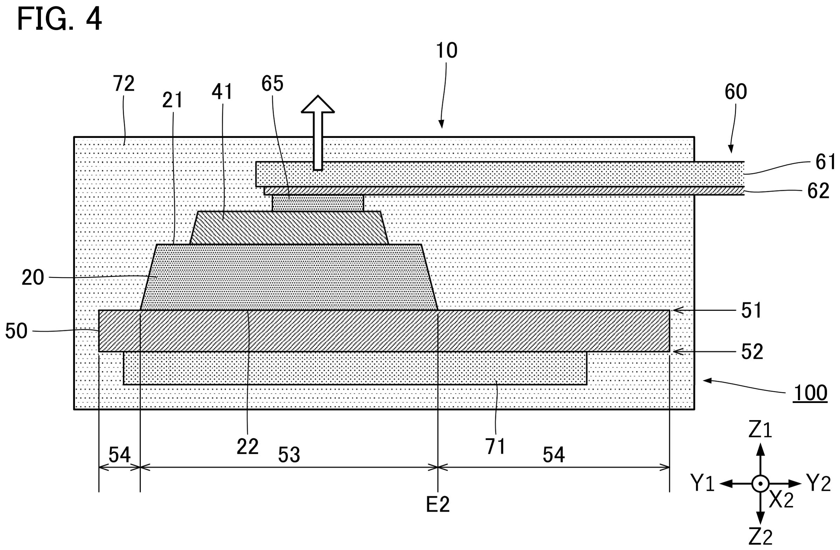

Fig. 4 is a cross-sectional view of a configuration (hereinafter referred to as a "comparative example") for use in contrast with the above-described embodiment. In the comparative example, the insulating layer 30, and, in each of the first electrodes 40, the connection portion 43 and the terminal portion 42 in the above-described embodiment are omitted.

In the comparative example, the wiring substrate 60 is bonded to the first surface 21 of the piezoelectric body 20 and the electrode portion 41 each of the plurality of first electrodes 40. As illustrated by the arrow in Fig. 4, an external force acting on the wiring substrate 60 in the Z1 direction may act on the first electrode 40 and the piezoelectric body 20. Accordingly, in the comparative example, there is a possibility that the first electrode 40 may be subject to peeling from the first surface 21 of the piezoelectric body 20 or the piezoelectric body may be subject to damage. For example, in a configuration in which the piezoelectric body 20 is constituted of stacked layers, the layers of the piezoelectric body 20 may peel due to an external force in the Z1 direction acting on the first surface 21.

In contrast to the comparative example, in the present embodiment described above, the wiring substrate 60 is bonded to each terminal portion 42 of the first electrodes 40 and to the peripheral edge portion 32 of the insulating layer 30. As seen in plan view, the peripheral edge portion 32 and the terminal portion 42 do not overlap the piezoelectric body 20. Accordingly, an external force in the Z1 direction does not act on the first surface 21 of the piezoelectric body 20 or the electrode portions 41 of the first electrodes 40 via the wiring substrate 60. That is, an external force acting on the piezoelectric body 20 via the wiring substrate 60 is minimized. Therefore, in contrast to the comparative example, according to the present embodiment, damage to the piezoelectric body 20 caused by an external force acting from the wiring substrate 60 can be prevented or reduced.

In the present embodiment, the line width Wb of each of the terminal portions 42 of the first electrodes 40 is smaller than the line width Wa of the electrode portion 41 each of the first electrodes 40. Accordingly, sufficient spacing Db can be secured between the terminal portions 42 of the first electrodes 40. Since sufficient spacing Db between the terminal portions 42 can be secured, a likelihood of short-circuiting of the terminal portions 42 of the first electrodes 40 is minimized.

For example, when the first electrodes 40 are each formed using a printing technique, there is a possibility that a conductive liquid material of the first electrodes 40 may spread in the X-axis direction in the stepped portion of the insulating layer 30 while wetting. Such spread may make adjacent first electrodes 40 prone to short-circuiting. In particular, as seen in plan view, a step is made in a portion (connection portion 43) of each of the first electrodes 40, and that the step overlaps the peripheral edge E1 of the insulating layer 30 or the peripheral edge E2 of the piezoelectric body 20. As a result, the connection portions 43 of adjacent first electrodes 40 are particularly susceptible to short-circuiting. According to the configuration in which the line width Wb of each of the terminal portions 42 is smaller than the line width Wa of a corresponding electrode portion 41, sufficient spacing Db between the terminal portions 42 can be secured, and thus susceptibility to short-circuiting of the first electrodes 40 is minimized.

### B: Manufacturing method

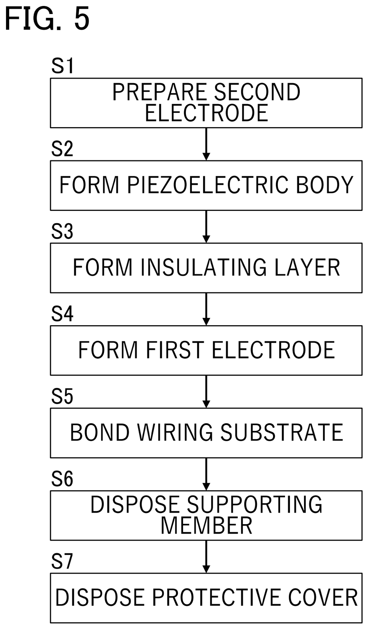

Fig. 5 is a flowchart showing steps of manufacturing the ultrasound probe 100. In the first step S1, the second electrode 50 is prepared. At step S2, after step S1 has been performed, the piezoelectric body 20 is formed on the front surface 51 of the second electrode 50. As described above, the piezoelectric body 20 is formed by, for example, the sol-gel method.

At step S3, after step S2 has been performed, the insulating layer 30 is formed over the first surface 21 of the piezoelectric body 20 and the peripheral edge portions 32 of the second electrode 50. At step S4, after step S3 has been performed, the first electrodes 40 are formed. Each of the first electrodes 40 extends in the Y-axis direction from the first surface 21 of the piezoelectric body 20 to the peripheral edge portion 32 of the insulating layer 30. By way of the above steps (S1 to S4), the piezoelectric unit 10 is manufactured.

At step S5, after step S4 has been completed, the wiring substrate 60 is bonded to the peripheral edge portion 32 of the insulating layer 30 and the terminal portion 42 of each of the plurality of first electrodes 40. At step S6, after step S5 has been completed, the support member 71 is bonded to the rear surface 52 of the second electrode 50. Step S6 of disposing the support member 71 may be performed at any point during processing from step S1 to step S5. At step S7, after step S6 is completed, the protective cover 72 is formed to accommodate the piezoelectric unit 10, the support member 71, and the part of the wiring substrate 60. The ultrasound probe 100 is manufactured by way of the above steps (S1 to S7).

### C: Modifications

Specific variations to the embodiment described above are exemplified below. Two or more aspects may be selected from the following examples and combined, as appropriate, in so far as no contradiction arises.
(1) In the above-described embodiment, the piezoelectric body 20 extends continuously over the first electrodes 40. However, the piezoelectric body 20 may be provided as separate bodies distanced from each other for each of the first electrodes 40 (that is, for each of the piezoelectric elements). Similarly, in the above-described embodiment, the insulating layer 30 extends continuously over the first electrodes 40. However, the insulating layer 30 may be provided separately for each of the first electrodes 40 (that is, for each of the piezoelectric elements).

In the above-described embodiment, since the piezoelectric body 20 extends continuously over the first electrodes 40, the manufacturing process of the ultrasound probe 100 can be simplified, as compared with a configuration in which the piezoelectric body 20 is provided as a separate body each for the first electrodes 40. Similarly, in the above-described embodiment, since the insulating layer 30 extends continuously over the first electrodes 40, the manufacturing process of the ultrasound probe 100 can be simplified, as compared with a configuration in which the insulating layer 30 is provided separately for each of the first electrodes 40.

In a configuration in which the piezoelectric body 20 is provided as separate bodies for each of the first electrodes 40, the insulating layer 30 is formed over both the first surfaces 21 each of piezoelectric bodies 20, and the gaps between the piezoelectric bodies 20. As a result, stress may concentrate at specific locations in the insulating layer 30 and cause damage to the insulating layer 30. In the above-described embodiment, since the piezoelectric body 20 and the insulating layer 30 are each provided to be continuous over the first electrodes 40, concentration of local stress in the insulating layer 30 is minimized, and a likelihood of damage to the insulating layer 30 is minimized.

(2) In the above embodiment, the line width Wb of the terminal portion 42 of each of the first electrodes 40 is less than the line width Wa of the corresponding electrode portion 41. However, the line width W of each of the first electrodes 40 may be made constant over the entire length of each of the first electrodes 40. For example, the line width Wa of the electrode portion 41 and the line width Wb of the terminal portion 42 (further, the line width Wc of the connection portion 43) may be the same. Similarly, the spacing Da between adjacent electrode portions 41 and the spacing Db between adjacent terminal portions 42 (further, the spacing Dc between adjacent connection portions 43) may be the same.

(3) Conditions such as dimensions, materials, or manufacturing methods for each element (the piezoelectric body 20, the insulating layer 30, the first electrode 40, the second electrode 50, the wiring substrate 60, the support member 71, and the protective cover 72) of the ultrasound probe 100 according to the embodiment are not limited to the examples described above, and may be freely changed.

(4) In the above-described embodiment there is a plurality of first electrodes 40. However, a single first electrode 40 may be provided. In such a case, the single first electrode 40 includes an electrode portion 41, a terminal portion 42, and a connection portion 43 as in the above-described embodiment. The planar shape of the single first electrode 40 may be freely changed. For example, the electrode portion 41 of the single first electrode 40 may be a circular shape or a polygonal shape.

(5) In the above-described embodiment the peripheral edge portion 32 of the insulating layer 30 is provided on the second electrode 50 (the overhang portion 54). However, an element on which the peripheral edge portion 32 of the insulating layer 30 is provided is not limited to the second electrode 50. For example, in a configuration in which the second electrode 50 or the piezoelectric body 20 is provided on the insulating substrate, the peripheral edge portion 32 of the insulating layer 30 may be provided on the surface of the insulating substrate.

(6) In the above-described embodiment, an example is given of the ultrasound probe 100 for use in observation of a living body. However, the use of the ultrasound probe 100 is not limited to the above example and may be freely changed. For example, the ultrasound probe 100 may be used for non-destructive inspection of a structure such as a building. An object (e.g., a living body or a structure) to be observed by the ultrasound probe 100 may be freely changed.

(7) The phrase "n" (n is a natural number) in the present application is used only as a formal and convenient label for distinguishing each element in the notation, and has no substantial meaning. Accordingly, a position of an element, order of manufacture, or the like cannot be restrictively limited on basis of the notation "n."

### D: Appendix

The following aspects are derivable from the embodiments above, for example.

An ultrasound probe according to an aspect (aspect 1) of the present disclosure includes: a piezoelectric body including a first surface; an insulating layer including a covering portion that covers a portion of the first surface and a peripheral edge portion that, in plan view, does not overlap the piezoelectric body; a first electrode including an electrode portion in contact with the first surface of the piezoelectric body and a terminal portion in contact with a surface of the peripheral edge portion of the insulating layer; and a wiring substrate bonded to the terminal portion of the first electrode and to the peripheral edge portion of the insulating layer. In this aspect, the wiring substrate is bonded to the terminal portion of the first electrode and the peripheral edge portion of the insulating layer. The peripheral edge portion and the terminal portion do not overlap the piezoelectric body. Accordingly, an external force acting on the wiring substrate does not act on the first surface of the piezoelectric body or the electrode portions of the first electrodes. That is, an external force acting on the piezoelectric body from the wiring substrate is minimized. Accordingly, as compared with a configuration in which the wiring substrate is bonded to the first surface of the piezoelectric body and the electrode portion of the first electrode, it is possible to prevent or minimize damage to the piezoelectric body caused by an external force acting from the wiring substrate.

In an example (aspect 2) of aspect 1, the ultrasound probe further includes a second electrode, the piezoelectric body further includes a second surface opposite the first surface, the second electrode includes an electrode portion in contact with the second surface of the piezoelectric body and an overhang portion extending in plan view beyond a peripheral edge of the piezoelectric body, and the peripheral edge portion of the insulating layer is in contact with the overhang portion. In aspect 2, the peripheral edge portion of the insulating layer is in contact with the overhang portion of the second electrode. Accordingly, the peripheral edge portion of the insulating layer is supported by the second electrode.

In an example (aspect 3) of aspect 2, the ultrasound probe further includes a protective cover that covers the piezoelectric body, the insulating layer, the first electrode, and the second electrode. According to aspect 3, the piezoelectric body, the insulating layer, the first electrode, and the second electrode are protected by the protective cover.

In an example (aspect 4) of any of aspects 1 to 3, the ultrasound probe comprises a plurality of first electrodes including the first electrode, the plurality of first electrodes is spaced apart from one another, each of the plurality of first electrodes includes an electrode portion in contact with the first surface of the piezoelectric body and the terminal portion in contact with the surface of the peripheral edge portion, and a line width of a respective terminal portion of each of the plurality of first electrodes is smaller than a line width of a corresponding electrode portion. In aspect 4, in each of the plurality of first electrodes, the line width of the terminal portion is smaller than the line width of the electrode portion. Accordingly, it is easy to secure sufficient spacing between the terminal portions of the first electrodes. By securing sufficient spacing between the terminal portions a likelihood of short-circuiting between the terminal portions of the first electrodes is minimized.

In an example (aspect 5) of aspect 4, each of the plurality of first electrodes includes a connection portion that connects the electrode portion and the terminal portion, the connection portion in each of the plurality of first electrodes overlaps, in plan view, a peripheral edge of the insulating layer located on the first surface of the piezoelectric body and a peripheral edge of the piezoelectric body that is covered by the insulating layer, and a line width of the connection portion of each of the plurality of first electrodes is smaller than a line width of the corresponding electrode portion. Since a step is formed in a portion of the first electrode that overlaps the peripheral edge of the insulating layer or the peripheral edge of the piezoelectric body in plan view, the connection portions of the first electrodes adjacent to each other are susceptible to short-circuiting. Since the line width of the connection portion overlapping the peripheral edge of the insulating layer and the peripheral edge of the piezoelectric body in each of the plurality of first electrodes is smaller than the line width of the corresponding electrode portion, this configuration is particularly effective in that the likelihood of short-circuiting of the connection portions can be reduced.

In an embodiment (aspect 6) of any of aspects 1 to 5, the ultrasound probe comprises a plurality of first electrodes including the first electrode, the piezoelectric body is continuous over the plurality of first electrodes, and the insulating layer is continuous over the plurality of first electrodes. According to aspect 6, since the piezoelectric body is continuous over the plurality of first electrodes, the manufacturing process of the ultrasound probe can be simplified as compared with a configuration in which the piezoelectric body is divided for each of the plurality of first electrodes. Similarly, since the insulating layer is continuous over the plurality of first electrodes, the manufacturing process of the ultrasound probe can be simplified as compared with a configuration in which the insulating layer is divided for each of the plurality of first electrodes. Further, in a configuration in which the piezoelectric body is divided for each of the plurality of first electrodes, the insulating layer is formed to cover the first surface of each piezoelectric body and the gaps between adjacent piezoelectric bodies. Consequently, stress may concentrate in localized regions of the insulating layer, potentially damaging the insulating layer. According to the configuration in which the piezoelectric body and the insulating layer are continuous over the plurality of first electrodes, concentration of local stress in the insulating layer is suppressed, and as a result, a possibility of damage to the insulating layer is minimized.

### Description of Reference Signs

100... ultrasound probe, 10... piezoelectric unit, 20... piezoelectric body, 21... first surface, 22... second surface, 30... insulating layer, 31... covering portion, 32... peripheral edge portion, 33... stepped portion, 40... first electrode, 41... electrode portion, 42... terminal portion, 43... connection portion, 50... second electrode, 51... front surface, 52... rear surface, 53... electrode portion, 54... overhang portion, 60... wiring substrate, 61... insulating substrate, 62... wiring, 63... wiring, 65... anisotropic conductive film, 71... support member, 72... protective cover.

## Claims

1. An ultrasound probe comprising:
a piezoelectric body including a first surface;
an insulating layer including a covering portion that covers a portion of the first surface and a peripheral edge portion that, in plan view, does not overlap the piezoelectric body;
a first electrode including an electrode portion in contact with the first surface of the piezoelectric body and a terminal portion in contact with a surface of the peripheral edge portion of the insulating layer; and
a wiring substrate bonded to the terminal portion of the first electrode and to the peripheral edge portion of the insulating layer.

2. The ultrasound probe according to claim 1, further comprising a second electrode, wherein:
the piezoelectric body further includes a second surface opposite the first surface,
the second electrode includes an electrode portion in contact with the second surface of the piezoelectric body and an overhang portion extending in plan view beyond a peripheral edge of the piezoelectric body, and
the peripheral edge portion of the insulating layer is in contact with the overhang portion.

3. The ultrasound probe according to claim 2, further comprising a protective cover that covers the piezoelectric body, the insulating layer, the first electrode, and the second electrode.

4. The ultrasound probe according to claim 1, wherein:
the ultrasound probe comprises a plurality of first electrodes including the first electrode,
the plurality of first electrodes is spaced apart from one another,
each of the plurality of first electrodes includes an electrode portion in contact with the first surface of the piezoelectric body and the terminal portion in contact with the surface of the peripheral edge portion, and
a line width of a respective terminal portion of each of the plurality of first electrodes is smaller than a line width of a corresponding electrode portion.

5. The ultrasound probe according to claim 4, wherein:
each of the plurality of first electrodes includes a connection portion that connects the electrode portion and the terminal portion,
the connection portion in each of the plurality of first electrodes overlaps, in plan view, a peripheral edge of the insulating layer located on the first surface of the piezoelectric body and a peripheral edge of the piezoelectric body that is covered by the insulating layer, and
a line width of the connection portion of each of the plurality of first electrodes is smaller than a line width of the corresponding electrode portion.

6. The ultrasound probe according to claim 1, wherein:
the ultrasound probe comprises a plurality of first electrodes including the first electrode,
the piezoelectric body is continuous over the plurality of first electrodes, and the insulating layer is continuous over the plurality of first electrodes.
